# EUROPEAN PATENT APPLICATION

(11) **EP 4 543 141 A1**
(43) Date of publication of application: **23.04.2025**
(21) Application number: 24206649.6
(22) Date of filing: 15.10.2024
(51) Int. Cl.: H05B 3/04, A24F 40/10, A24F 40/46, H05B 3/14, H05B 3/26

(54) **HEATING ELEMENT AND PREPARATION METHOD THEREFOR, ATOMIZER, AND ELECTRONIC ATOMIZATION DEVICE**

(30) Priority: 20.10.2023 CN 202311369677
(71) Applicant: Shenzhen Smoore Technology Limited, Shenzhen, Guangdong 518102 (CN)
(72) Inventor: ZHANG, Zhao, Shenzhen, 518102 (CN); WANG, Ting, Shenzhen, 518102 (CN); TANG, Junjie, Shenzhen, 518102 (CN); LUO, Hongliang, Shenzhen, 518102 (CN); JIANG, Zhenlong, Shenzhen, 518102 (CN); XIAO, Congwen, Shenzhen, 518102 (CN)
(74) Representative: Herrero & Asociados, S.L.

(57) **Abstract**

A heating element and a preparation method therefor, an atomizer, and an electronic atomization device are disclosed. The heating element includes: a porous substrate, a heat dissipation layer, and a heating layer. The heat dissipation layer is disposed on the porous substrate, where the heat dissipation layer has a porous structure; and the heating layer is disposed on a surface of the heat dissipation layer away from the porous substrate.

## Description

### TECHNICAL FIELD

The present disclosure relates to the field of electronic atomization technologies, and in particular, to a heating element and a preparation method therefor, an atomizer, and an electronic atomization device.

### BACKGROUND

An electronic atomization device is formed by components such as a heating element, a battery, and a control circuit. The heating element is a core component of the electronic atomization device, and characteristics thereof determine an atomization effect and use experience of the electronic atomization device.

After being used for a specific quantity of times, an existing heating element has obvious fouling, affecting a sense of use experience of a user.

### SUMMARY

This application provides a heating element and a preparation method therefor, an atomizer, and an electronic atomization device, to improve a phenomenon of fouling of the heating element, and improve a sense of use experience of a user.

In order to resolve the technical problem, a first technical solution adopted in this application is to provide a heating element, including a porous substrate, a heat dissipation layer, and a heating layer. The heat dissipation layer is disposed on the porous substrate, where the heat dissipation layer has a porous structure; and the heating layer is disposed on a surface of the heat dissipation layer away from the porous substrate.

In an implementation, a thickness of the heat dissipation layer is less than a thickness of the heating layer; and/or a porosity of the heat dissipation layer is greater than a porosity of the heating layer; and/or the heat dissipation layer and the heating layer each have a strip-shaped structure, and a line width of the heat dissipation layer is greater than a line width of the heating layer.

In an implementation, the porosity of the heat dissipation layer is in a range of 30% to 70%; and/or the line width of the heat dissipation layer is in a range of 0.5 mm to 0.8 mm; and/or a resistance of the heat dissipation layer is in a range of 8 S2 to 20 Q; and/or the thickness of the heating layer is in a range of 40 µm to 100 µm; and/or the porosity of the heating layer is in a range of 0 to 10%; and/or the line width of the heating layer is in a range of 0.25 mm to 0.45 mm; and/or a resistance of the heating layer is in a range of 0.85 S2 to 1.60 Ω.

In an implementation, an orthographic projection of the heat dissipation layer on the heating layer completely covers the heating layer.

In an implementation, an orthographic projection of a center line of the heat dissipation layer along an extension direction on the heating layer coincides with a center line of the heating layer along the extension direction.

In an implementation, the porous substrate includes an atomization surface; the heat dissipation layer includes a main body portion and an infiltration portion, the main body portion is disposed on the atomization surface of the porous substrate, and the infiltration portion is disposed in holes of the porous substrate.

In an implementation, a thickness of the infiltration portion is in a range of 80 µm to 150 µm; and/or a thickness of the main body portion is in a range of 5 µm to 20 µm.

In an implementation, a material of the heating layer includes at least one of a nickel-based alloy, an iron-based alloy, and a ruthenium-based alloy; and/or a material of the heat dissipation layer includes at least one of the nickel-based alloy, the iron-based alloy, and the ruthenium-based alloy.

In an implementation, the heating layer and the heat dissipation layer share the same shape and both extend in a curve.

In an implementation, a line length of the heat dissipation layer is the same as a line length of the heating layer.

To resolve the foregoing technical problem, a second technical solution provided in this application is to provide an atomizer, including a liquid storage cavity and the heating element of any one of the foregoing, where the liquid storage cavity is configured to store an aerosol-forming material, and the heating element is configured to heat and atomize the aerosol-forming material in the liquid storage cavity.

To resolve the foregoing technical problem, a third technical solution provided in this application is to provide an electronic atomization device, including a main unit and the atomizer, where the main unit is configured to supply power to the atomizer.

To resolve the foregoing technical problem, a fourth technical solution provided in this application is to provide a preparation method for a heating element, including: providing a porous substrate, where the porous substrate includes an atomization surface; coating a first paste on the atomization surface of the porous substrate, to form a heat dissipation layer; and coating a second paste on a surface of the heat dissipation layer, to form a heating layer.

In an implementation, a viscosity of the first paste is less than a viscosity of the second paste.

In an implementation, the viscosity of the first paste is in a range of 50000 cp to 150000 cp; and/or the viscosity of the second paste is in a range of 400000 cp to 800000 cp.

The beneficial effects of this application are as follows. Different from the related art, this application provides a heating element and a preparation method therefor, an atomizer, and an electronic atomization device. The heating element includes a porous substrate, a heat dissipation layer, and a heating layer. The heat dissipation layer is disposed on the porous substrate, where the heat dissipation layer has a porous structure; and the heating layer is disposed on a surface of the heat dissipation layer away from the porous substrate. The heat dissipation layer is disposed to have the porous structure, so that a liquid supply channel is provided to the heating layer, to maintain a sufficient liquid supply amount to the heating layer and continuous atomization. The heat dissipation layer is disposed between the porous substrate and the heating layer, so that the heat dissipation layer in contact with the porous substrate can quickly conduct heat to a region of the porous substrate that is not covered by the heat dissipation layer, and an aerosol-forming material stored in the region of the porous substrate that is not covered by the heat dissipation layer can be quickly atomized. Based on a fact that the same amount of atomization is implemented, temperature field distribution of the atomization surface of the heating element can be improved and liquid supply can be more efficient, and a phenomenon of fouling is improved, to improve a sense of use experience of a user.

### BRIEF DESCRIPTION OF THE DRAWINGS

FIG. 1 is a schematic diagram of a structure of an electronic atomization device according to an embodiment of this application.
FIG. 2 is a schematic diagram of a structure of a heating element according to an embodiment of this application.
FIG. 3 is a schematic diagram of an exploded structure of the heating element shown in FIG. 2.
FIG. 4 is a partially enlarged schematic diagram of a heat dissipation layer and a porous substrate according to an embodiment of this application.
FIG. 5 is a flowchart of a preparation method for a heating element according to an embodiment of this application.
FIG. 6 is a schematic diagram of a product of a first experimental member.
FIG. 7 is a schematic diagram of a product of a second experimental member.
FIG. 8 is a schematic diagram of a product of a third experimental member.
FIG. 9 is a comparison diagram of fouling of a first experimental member, a second experimental member, and a third experimental member after 500 puffs using a material 1.
FIG. 10 is a comparison diagram of temperature fields of a first experimental member, a second experimental member, and a third experimental member.
FIG. 11 is a curve diagram of highest temperatures of a first experimental member, a second experimental member, and a third experimental member.
FIG. 12 is a comparison diagram of infiltration of a first experimental member and a second experimental member.
FIG. 13 is a comparison diagram of fouling after inhaling of a first experimental member, a second experimental member, and a third experimental member.
FIG. 14 is a comparison diagram of a degree of flatness of a first experimental member, a second experimental member, and a third experimental member.
FIG. 15 is a flowchart of a preparation product of a second experimental member.

Descriptions of reference numerals:
1-Atomizer; 2-main unit; 10-heating element; 11-porous substrate; 111-atomization surface; 112-liquid absorbing surface; 12-heat dissipation layer; 121-main body portion; 122-infiltration portion; and 13-heating layer.

### DETAILED DESCRIPTION

The technical solutions in the embodiments of this application are clearly and completely described in the following with reference to the accompanying drawings in the embodiments of this application. Apparently, the described embodiments are merely some rather than all of the embodiments of this application. All other embodiments obtained by a person of ordinary skill in the art based on embodiments of this application without creative efforts shall fall within the protection scope of this application.

In this application, the terms "first", "second" and "third" are used merely for the purpose of description, and shall not be construed as indicating or implying relative importance or implying a quantity of indicated technical features. Therefore, features defining "first", "second", and "third" can explicitly or implicitly include at least one of the features. In description of this application, "more" means at least two, such as two and three unless it is specifically defined otherwise. All directional indications (for example, up, down, left, right, front, back) in the embodiments of this application are only used for explaining relative position relationships, movement situations, or the like between the various components in a specific posture (as shown in the accompanying drawings). If the specific posture changes, the directional indications change accordingly. In addition, the terms "including" and "having" and any other variants thereof are intended to cover a non-exclusive inclusion. For example, a process, a method, a system, a product, or a device that includes a series of steps or units is not limited to the listed steps or units, but optionally further includes an unlisted step or unit, or optionally further includes another inherent step or unit of the process, the method, the product, or the device.

"Embodiment" mentioned in the specification means that particular features, structures, or characteristics described with reference to the embodiment may be included in at least one embodiment of this application. The term appearing at different positions of the specification may not refer to the same embodiment or an independent or alternative embodiment that is mutually exclusive with another embodiment. A person skilled in the art explicitly or implicitly understands that the embodiments described in the specification may be combined with other embodiments.

This application is described in detail below with reference to the accompanying drawings and embodiments.

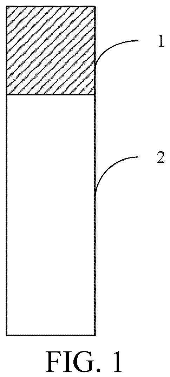

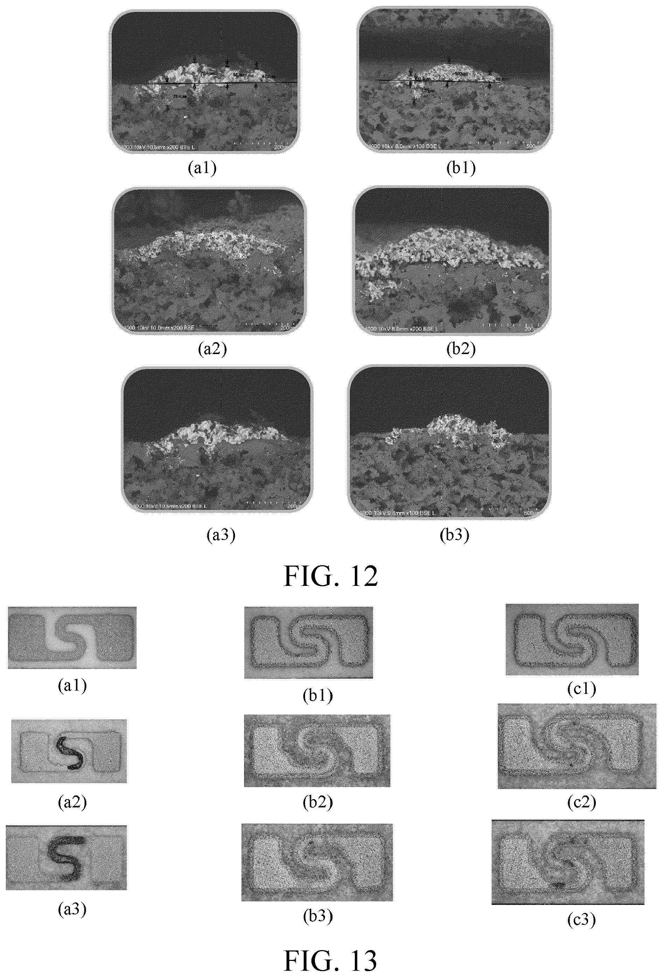

FIG. 1 is a schematic diagram of a structure of an electronic atomization device according to an embodiment of this application.

In this embodiment, an electronic atomization device is provided. The electronic atomization device may be configured to atomize an aerosol-forming material. The electronic atomization device includes an atomizer 1 and a main unit 2 that are electrically connected to each other.

The atomizer 1 includes a liquid storage cavity and a heating element 10. The liquid storage cavity is configured to store the aerosol-forming material, and the heating element 10 is configured to heat and atomize the aerosol-forming material in the liquid storage cavity to form aerosols that can be inhaled by a user. The atomizer 1 specifically may be applied to different fields such as medical care, cosmetology, and recreation inhalation. In a specific embodiment, the atomizer 1 may be applied to an electronic aerosol atomization device to atomize the aerosol-forming material and generate aerosols for inhalation by an inhaler, and the following embodiments are described by using the recreation inhalation as an example.

For a structure and a function of the atomizer 1 other than the heating element 10, refer to the related art. Details are not described herein again.

The main unit 2 includes a power supply (not shown in the figure) and a controller (not shown in the figure). The power supply is configured to supply electric energy to operation of the atomizer 1, to cause the atomizer 1 to atomize the aerosol-forming material to form aerosols; and the controller is configured to control the atomizer 1 to operate. The main unit 2 further includes other components such as a battery holder and an airflow sensor.

The atomizer 1 and the main unit 2 may be integrally disposed or may be detachably connected to each other, which may be designed according to a specific requirement.

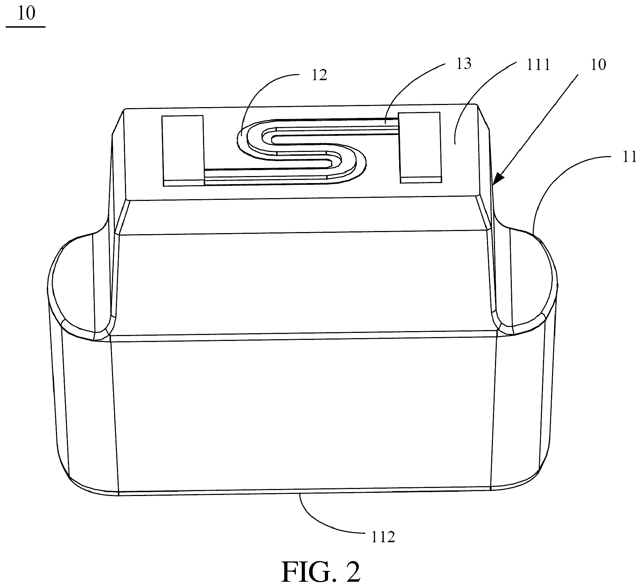

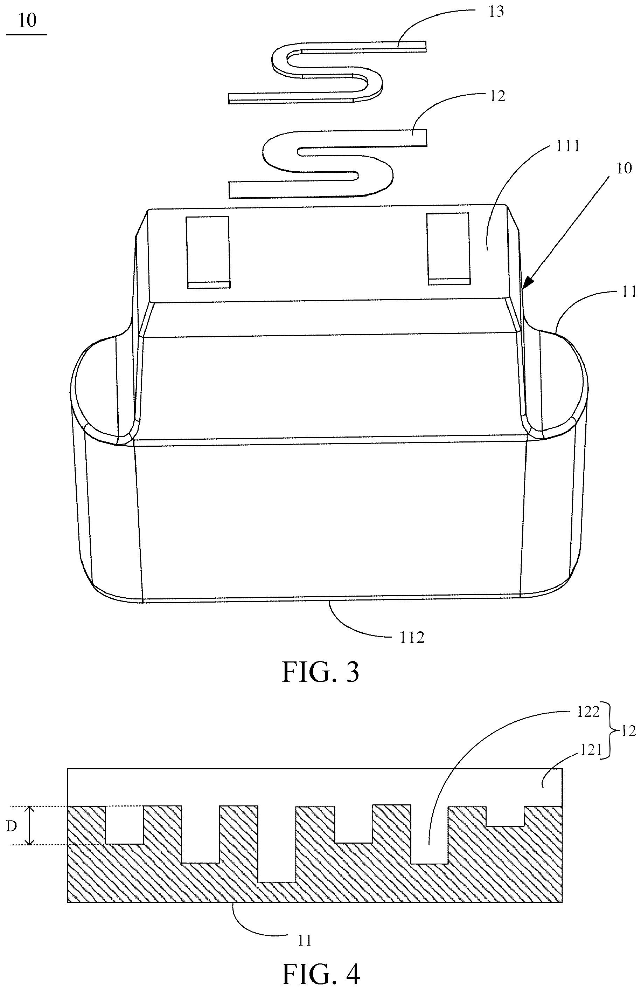

FIG. 2 is a schematic diagram of a structure of a heating element according to an embodiment of this application, and FIG. 3 is a schematic diagram of an exploded structure of the heating element shown in FIG. 2.

The heating element 10 includes a porous substrate 11, a heat dissipation layer 12, and a heating layer 13.

The porous substrate 11 has an atomization surface 111 and a liquid absorbing surface 112 that are oppositely disposed. The porous substrate 11 has a porous structure, and the porous structure has a capillarity force. A surface of the porous substrate 11 close to a liquid storage cavity is the liquid absorbing surface 112. An aerosol-forming material on the liquid absorbing surface 112 is guided to the atomization surface 111 by the capillarity force of the porous substrate 11. In this implementation, the porous substrate 11 is a porous ceramic. The porous ceramic forms disordered holes that are in communication with each other in a preparation process, and has the capillarity force. In other implementations, the porous substrate 11 has another porous structure. For example, a plurality of through holes are provided on a dense substrate to form the porous structure. The dense substrate may be a dense ceramic, glass, or the like.

The heat dissipation layer 12 is disposed on the porous substrate 11. The heat dissipation layer 12 has the porous structure. The heat dissipation layer 12 is in contact with the porous substrate 11. In an implementation, the heat dissipation layer 12 is formed by coating a first paste on a surface of the atomization surface 111. The formed heat dissipation layer 12 has the porous structure. A manner of forming the heat dissipation layer 12 having the porous structure is simple and easy, and does not damage a structure of the porous substrate 11.

The heating layer 13 is disposed on a surface of the heat dissipation layer 12 away from the porous substrate 11. The heat dissipation layer 12 has the porous structure, so that a liquid supply channel is provided to the heating layer 13 disposed on the surface of the heat dissipation layer 12, to maintain a sufficient liquid supply amount to the heating layer 13 and a continuous atomization. The heat dissipation layer 12 is disposed between the porous substrate 11 and the heating layer 13. The heat dissipation layer 12 can fill defects on the surface of the porous substrate 11. The heat dissipation layer 12 provides a flatter surface than the porous substrate 11, and the heating layer 13 is formed on the flatter surface. An impact of defects on the surface of the porous substrate 11 on a resistance value of the heating layer 13 is reduced, and consistency of the resistance value of the heating layer 13 is improved.

In this implementation, heat conductivity of the heat dissipation layer 12 is good, and a heat transfer rate of the heat dissipation layer 12 is higher than a heat transfer rate of the porous substrate 11, so that the heat dissipation layer 12 in contact with the porous substrate 11 can quickly conduct heat to a region of the porous substrate 11 that is not covered by the heat dissipation layer 12, and an aerosol-forming material stored in the region of the porous substrate 11 that is not covered by the heat dissipation layer 12 can be quickly atomized. Based on a fact that the same amount of atomization is implemented, an atomization temperature of the heating element 10 can be reduced, a national standard is satisfied, and a sense of use experience of a user is improved. After analysis, the inventor considers that, to achieve the foregoing effects, the following aspects are mainly to be performed for improvement: First, the heat dissipation layer 12 uses a material with a higher heat conducting coefficient, and the material partially fills holes or defects of the porous substrate 11 corresponding to a region of the atomization surface, thereby improving a heat conducting capability of the part, that is, a heat conducting coefficient of the region is further increased; second, disposing of the heat dissipation layer 12 increases a binding depth between the heating layer 13 and the porous substrate 11, so that heat can be quickly conducted over a larger range, and a heat resistance is also reduced; and third, the heat dissipation layer 12 has a great porosity, and the aerosol-forming material also has good flowability in the region, to avoid high-temperature dry heating caused by lack of liquid.

In an atomization process, the heat dissipation layer 12 mainly plays a role of quick heat conduction, and the heating layer 13 mainly plays a role of atomizing the aerosol-forming material.

FIG. 4 is a partially enlarged schematic diagram of a heat dissipation layer and a porous substrate according to an embodiment of this application.

The heat dissipation layer 12 includes a main body portion 121 and an infiltration portion 122, the main body portion 121 is disposed on the atomization surface 111 of the porous substrate 11, and the infiltration portion 122 is disposed in holes of the porous substrate 11. In an implementation, in a process of coating a first paste on a surface of the porous substrate 11 to form the heat dissipation layer 12, a part of the first paste is formed on the atomization surface 111 of the porous substrate 11 to form the main body portion 121, and a part of the first paste infiltrates into the holes of the porous substrate 11 to form the infiltration portion 122. In this case, the thickness of the infiltration portion 122 is related to a viscosity of the first paste (where the viscosity of the first paste affects flowability of the first paste, further affects a depth at which the first paste infiltrates into the holes, and affects the thickness of the infiltration portion 122 formed by the first paste in the holes). The infiltration portion 122 of the heat dissipation layer 12 is disposed in the holes of the porous substrate 11, which improves a binding force between the heat dissipation layer 12 and the porous substrate 11, greatly reduces a risk of disengagement and tilt of the heat dissipation layer 12, and improves a service life of the heating element 10.

The thickness D of the infiltration portion 122 is in a range of 80 µm to 150 µm, so that the heat dissipation layer 12 has a strong binding force with the porous substrate 11. The thickness D of the infiltration portion 122 may be 80 µm, 90 µm, 100 µm, 110 µm, 120 µm, 130 µm, 140 µm, 150 µm, or the like, or may be a range formed by any two of these values. The thickness D of the infiltration portion 122 is a distance from a connection point between the infiltration portion 122 and the main body portion 121 to an endpoint of the infiltration portion 122 farthest from the main body portion 121.

It should be noted that, FIG. 4 only shows a partial hole structure of the porous substrate 11. The thickness of the infiltration portion 122 in different holes of the porous substrate 11 may be the same or different. For example, the porous substrate 11 is a porous ceramic. The porous ceramic has a plurality of disordered holes formed in a preparation process. The disordered holes are irregular. During coating of the first paste, depths at which the first paste infiltrating into different disordered holes are different. As a result, the thicknesses of different infiltration portions 122 are somewhat the same and are somewhat different.

In an implementation, the thickness (namely, the thickness of the main body portion 121 of the heat dissipation layer 12) of a part of the heat dissipation layer 12 disposed on a surface of the porous substrate 11 is less than the thickness of the heating layer 13. Because the thickness of the part of the heat dissipation layer 12 disposed on the surface of the porous substrate 11 is relatively small relative to the thickness of the heating layer 13, heat generated by the heat dissipation layer 12 and heat transferred to the heat dissipation layer 12 by the heating layer 13 are easier to be transmitted outward, so that the heat dissipation layer 12 mainly plays a role of quick heat conduction.

Optionally, the thickness of the main body portion 121 of the heat dissipation layer 12 is in a range of 5 µm to 20 µm. The thickness of the main body portion 121 of the heat dissipation layer 12 may be 5 µm, 10 µm, 15 µm, 20 µm, or the like, or may be a range formed by any two of these values. When the thickness of the main body portion 121 of the heat dissipation layer 12 is less than 5 µm, an effect of covering defects on the surface of the porous substrate 11 is not good. As a result, the heating layer 13 cannot be provided with a relatively flat surface. When the thickness of the main body portion 121 of the heat dissipation layer 12 is greater than 20 µm, a heat dissipation effect and a heat conducting effect are poor.

Optionally, the thickness of the heating layer 13 is in a range of 40 µm to 80 µm; and the thickness of the heating layer 13 may be 40 µm, 60 µm, 80 µm, 100 µm, or the like, or may be a range formed by any two of these values. The thickness of the heating layer 13 is set to be in a range of 40 µm to 80 µm, so that the heating layer 13 has high atomization efficiency at low energy consumption.

In an implementation, a porosity of the heat dissipation layer 12 is greater than a porosity of the heating layer 13. Because the porosity of the heat dissipation layer 12 is greater than the porosity of the heating layer 13, heat generated by the heat dissipation layer 12 and heat transferred to the heat dissipation layer 12 by the heating layer 13 are easier to be transmitted outward, so that the heat dissipation layer 12 mainly plays a role of quick heat conduction. In addition, the porosity of the heating layer 13 is less than the porosity of the heat dissipation layer 12. In an atomization process, the heating layer 13 has less stored liquid. Under the same heating power, the stored liquid can be quickly heated and atomized, to avoid formation of a thicker liquid film on the surface of the heating layer 13, avoiding a phenomenon of liquid explosion caused by the thicker liquid film in the atomization process.

Optionally, the porosity of the heat dissipation layer 12 is in a range of 30% to 70%; and the porosity of the heat dissipation layer 12 may be 30%, 50%, 70%, or the like, or may be a range formed by any two of these values. The porosity of the heat dissipation layer 12 is set to be in a range of 30% to 70%, and a liquid supply amount of the heat dissipation layer 12 matches an atomization capability of the heating layer 13, to maintain a sufficient liquid supply amount of the heating layer 13. When the porosity of the heat dissipation layer 12 is less than 30%, it is prone to a problem that the liquid supply amount of the heat dissipation layer 12 is small, atomization efficiency of the heating layer 13 is low, and even dry heating occurs. When the porosity of the heat dissipation layer 12 is greater than 70%, it is prone to a problem that the liquid supply amount of the heat dissipation layer 12 is large, and the heating layer 13 cannot atomize the liquid in a timely manner, resulting in a problem of liquid leaking.

Optionally, the porosity of the heating layer 13 is in a range of 0 to 10%; and the porosity of the heating layer 13 may be 0, 3%, 6%, 9%, 10%, or the like, or may be a range formed by any two of these values. When the porosity of the heating layer 13 is 0, the heating layer 13 has a dense structure. When the porosity of the heating layer 13 is greater than 0, the heating layer 13 has a porous structure. When the heating layer 13 has the porous structure, wettability of the aerosol-forming material on the entire heating layer 13 may be improved, facilitating rapid atomization of the aerosol-forming material, and improving atomization efficiency of the heating element 10. The porosity of the heating layer 13 is greater than 10%, consistency of a resistance value of the heating layer 13 is difficult to control, and consistency of the heating element 10 is poor.

In an implementation, the heat dissipation layer 12 and the heating layer 13 each have a strip-shaped structure, and the line width of the heat dissipation layer 12 is greater than the line width of the heating layer 13. Because the line width of the heat dissipation layer 12 is wide relative to the line width of the heating layer 13, the heat dissipation layer 12 has a large contact area with the porous substrate 11, and heat generated by the heat dissipation layer 12 and heat transferred to the heat dissipation layer 12 by the heating layer 13 are easier to be transmitted outward, so that the heat dissipation layer 12 mainly plays a role of quick heat conduction.

Optionally, the line width of the heat dissipation layer 12 is in a range of 0.5 mm to 0.8 mm; and the line width of the heat dissipation layer 12 may be 0.5 mm, 0.6 mm, 0.7 mm, 0.8 mm, or the like, or may be a range formed by any two of these values. The line width of the heat dissipation layer 12 is set to be in a range of 0.5 mm to 0.8 mm, to implement a good heat conducting effect. The heat dissipation layer 12 considers both heat field distribution and a liquid supply capability, to minimize fouling on the surface of the heating layer 13, thereby reducing interference of the fouling on a heating function of the heating layer 13, and improving consistency of an atomization amount. The heat dissipation layer 12 has a good heat conducting effect, so that the heat dissipation layer 12 and a region around the heating layer 13 also have a quick consumption rate of the aerosol-forming material, and fewer aerosol-forming materials around the heating layer 13 flow in a direction of the heating layer 13. In this way, fewer flowing aerosol-forming materials that carry fouling generated in the region around the heating layer 13 deposit on the heating layer 13, so that fouling of the heating layer 13 is reduced.

Optionally, the line width of the heating layer 13 is in a range of 0.25 mm to 0.45 mm; and the line width of the heating layer 13 may be 0.25 mm, 0.3 mm, 0.35 mm, 0.4 mm, 0.45 mm, or the like, or may be a range formed by any two of these values. The line width of the heating layer 13 is set to be in a range of 0.25 mm to 0.45 mm, so that there is a large heating region, and a good atomization effect is implemented.

In an implementation, a resistance of the heat dissipation layer 12 is in a range of 8 S2 to 20 Ω; and the resistance of the heat dissipation layer 12 may be 8 S2, 10 S2, 12 S2, 14 S2, 16 S2, 18 S2, 20 S2, or the like, or may be a range formed by any two of these values. It should be noted that, the foregoing parameters may be used in the thickness and/or the porosity and/or the line width of the heat dissipation layer 12, so that the resistance of the heat dissipation layer 12 is in a range of 8 S2 to 20 Ω.

In an implementation, a resistance of the heating layer 13 is in a range of 0.85 S2 to 1.60 Ω; and the resistance of the heating layer 13 may be 0.85 S2, 1.05 S2, 1.25 S2, 1.45 S2, 1.60 S2, or the like, or may be a range formed by any two of these values. It should be noted that, the foregoing parameters may be used in the thickness and/or the porosity and/or the line width of the heating layer 13, so that the resistance of the heating layer 13 is in a range of 0.85 S2 to 1.60 Ω.

The foregoing design is applied to the resistance of the heat dissipation layer 12 and the resistance of the heating layer 13, that is, the resistance of the heat dissipation layer 12 is much greater than the resistance of the heating layer 13, so that the heat dissipation layer 12 is mainly configured for quick heat conductivity, and the heating layer 13 is mainly configured for heating. Heat of the heating layer 13 is transferred to the heat dissipation layer 12. A quick temperature dissipation effect of the heat dissipation layer 12 is used, so that temperature field performance is stable and consistent, is lower than 300 °C, and fully satisfies the national standard.

In an implementation, a material of the heating layer 13 includes at least one of a nickel-based alloy, an iron-based alloy, and a ruthenium-based alloy; and/or a material of the heat dissipation layer 12 includes at least one of the nickel-based alloy, the iron-based alloy, and the ruthenium-based alloy. It may be understood that, the material of the heat dissipation layer 12 and the material of the heating layer 13 can be the same or different. Optionally, the material of the heat dissipation layer 12 is doped with a small amount of ceramic powder, to improve a binding force between the heat dissipation layer 12 and the porous substrate 11 (in this case, the porous substrate 11 is a porous ceramic), and improve a heat transfer effect of the heat dissipation layer 12 to the porous substrate 11.

In an implementation, an orthographic projection of the heat dissipation layer 12 on the heating layer 13 completely covers the heating layer 13. The heat dissipation layer 12 can quickly conduct heat generated by the heating layer 13. A quick temperature dissipation effect of the heat dissipation layer 12 is used, so that temperature field performance is stable and a temperature is low.

Optionally, as shown in FIG. 2, an orthographic projection of a center line of the heat dissipation layer 12 along an extension direction on the heating layer 13 coincides with a center line of the heating layer 13 along the extension direction. In this case, an amount of liquid supplied by the heat dissipation layer 12 to the heating layer 13 is basically consistent, and the heating layer 13 has high atomization efficiency.

Optionally, the heating layer 13 is biased on the heat dissipation layer 12. In other words, the orthographic projection of the center line of the heat dissipation layer 12 along the extension direction on the heating layer 13 is offset from the center line of the heating layer 13 along the extension direction, and the orthographic projection of the heat dissipation layer 12 on the heating layer 13 completely covers the heating layer 13.

The center line of the heat dissipation layer 12 extends along an extension direction from a middle point at one end of the heat dissipation layer 12 to a middle point at the other end of the heat dissipation layer 12. Any point on the center line of the heat dissipation layer 12 is at an equal vertical distance from edges of the heat dissipation layer 12 on two sides of the heat dissipation layer 12. The center line of the heating layer 13 is defined similarly to the center line of the heat dissipation layer 12.

In an implementation, a shape of the heating layer 13 is the same as a shape of the heat dissipation layer 12. Both the heating layer 13 and the heat dissipation layer 12 extend in a curve, to increase a contact area between the heat dissipation layer 12 and the porous substrate 11 within an effective area of an atomization surface 111.

Optionally, the heat dissipation layer 12 extends in an "S" shaped curve. Specifically, the heat dissipation layer 12 includes a first straight line segment, a second straight line segment, a third straight line segment, a first curve segment, and a second curve segment. The first straight line segment, the second straight line segment, and the third straight line segment are parallel to each other, and the second straight line segment is located between the first straight line segment and the third straight line segment. The first curve segment connects the first straight line segment and the second straight line segment, and the first straight line segment, the first curve segment, and the second straight line segment are sequentially connected end to end. The second curve segment connects the second straight line segment and the third straight line segment, and the second straight line segment, the second curve segment, and the third straight line segment are sequentially connected end to end. The heating layer 13 also extends in an "S" shaped curve, and a specific setting manner of the heating layer 13 is similar to the structure of the heat dissipation layer 12.

In an implementation, the line length of the heat dissipation layer 12 is the same as the line length of the heating layer 13.

It should be noted that, the heat dissipation layer 12 and the heating layer 13 are sequentially disposed on the surface of the porous substrate 11, so that possibilities of adaptability of the heating element 10 to different aerosol-forming materials (hereinafter referred to as materials) are expanded. Through research, it is found that in a process of atomizing aerosol-forming materials such as a material 1 (for example, the crane dancing in the blue sky) and a material 2 (for example, aloe vera grape) by using the heating element 10 provided in the embodiments of this application, the higher the heating power, the better the heat conducting effect and the better the heat dissipation effect. In a test condition ranging from 8 W to 12 W, the highest-temperature field is basically stable at around 280 °C. Compared with the existing highest-temperature field at a range of 300 °C to 350 °C, the atomization temperature of the heating element 10 is reduced, which can satisfy the national standard. At a lower temperature, there is a better atomization effect, and the heating element 10 has a longer service life and a richer taste.

According to the heating element 10 provided in this embodiment, the service life of the heating element 10 is increased, and performance of the heat dissipation layer 12 and the heating layer 13 is improved. The heat dissipation layer 12 and the heating layer 13 with different resistance values are used, so that the temperature field performance is stable, and is lower than 300°C, a risk of a high temperature is reduced, and the national standard is satisfied. Two-layer design increases a product design space, so that different tastes can be adjusted, and an application space of the heating element 10 increases. In the embodiments of this application, an implementation is simple, and a significant effect is achieved. Product performance is significantly improved, and product application can be quickly implemented.

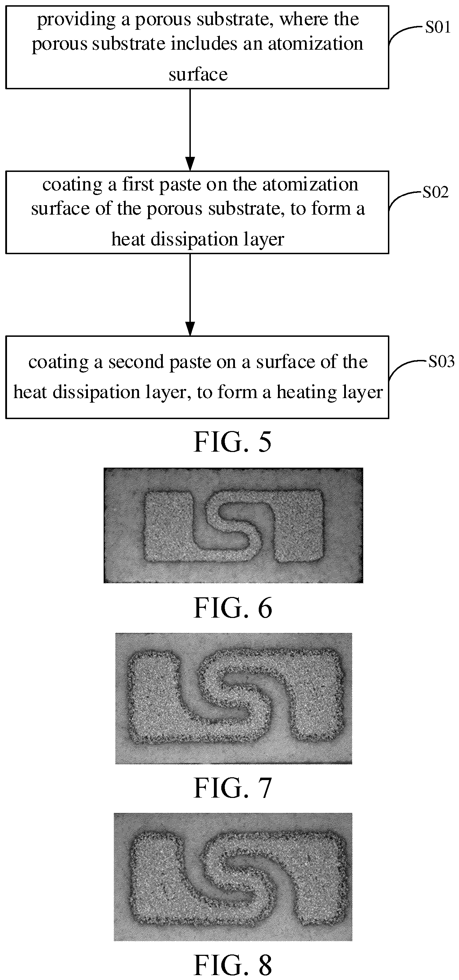

FIG. 5 is a flowchart of a preparation method for a heating element according to an embodiment of this application.

Embodiments of this application further provide a preparation method for a heating element. The preparation method for a heating element may be used to prepare the heating element 10 provided in the foregoing embodiments.

The preparation method for a heating element includes the following operations.

Operation S01: providing a porous substrate, where the porous substrate includes an atomization surface.

Specifically, the porous substrate 11 has an atomization surface 111 and a liquid absorbing surface 112 that are oppositely disposed. The porous substrate 11 has a porous structure, and the porous structure has a capillarity force. A surface of the porous substrate 11 close to a liquid storage cavity is the liquid absorbing surface 112. The aerosol-forming material on the liquid absorbing surface 112 is guided to the atomization surface 111 by the capillarity force of the porous substrate 11. In this implementation, the porous substrate 11 is a porous ceramic. The porous ceramic forms disordered holes that are in communication with each other in a preparation process, and has the capillarity force. In other implementations, the porous substrate 11 has another porous structure. For example, a plurality of through holes are provided on a dense substrate to form a porous structure. The dense substrate may be a dense ceramic, glass, or the like.

Operation S02: coating a first paste on the atomization surface of the porous substrate, to form a heat dissipation layer.

In an implementation, a viscosity of the first paste is in a range of 50000 cp to 150000 cp; and the viscosity of the first paste may be 50000 cp, 100000 cp, 150000 cp, or the like, or may be a range formed by any two of these values. The first paste includes at least one of a nickel-based alloy paste, an iron-based alloy paste, and a ruthenium-based alloy paste; and optionally, the first paste is doped with a small amount of ceramic powder.

The viscosity of the first paste is set to be in a range of 50000 cp to 150000 cp, which has a low viscosity and high flowability, so that a part of the first paste infiltrates the inside of holes of the porous substrate 11. A heat dissipation layer 12 is formed by coating the first paste. During formation, a part of the first paste infiltrates into the holes of the porous substrate 11, and a part of the first paste that infiltrates the inside of the porous substrate 11 forms an infiltration portion 122. A part of the first paste that does not infiltrate the inside of the porous substrate 11 and that is formed on the surface of the porous substrate 11 is a main body portion 121. In this way, the heat dissipation layer 12 has a strong binding force with the porous substrate 11, which greatly reduces a risk of disengagement and tilt of the heat dissipation layer 12, and increases a service life of the heating element 10. In an implementation, the thickness D of the infiltration portion 122 is in a range of 80 µm to 150 µm, so that the heat dissipation layer 12 has a strong binding force with the porous substrate 11. The thickness D of the infiltration portion 122 may be 80 µm, 90 µm, 100 µm, 110 µm, 120 µm, 130 µm, 140 µm, 150 µm, or the like, or may be a range formed by any two of these values.

In an implementation, a leveling time period of the first paste is extended or a pressing process is increased, so that a depth at which the first paste infiltrates the inside of the holes of the porous substrate 11 is increased. That is, the thickness D of the infiltration portion 122 is increased, and there is a strong binding force between the heat dissipation layer 12 and the porous substrate 11.

Operation S03: coating a second paste on a surface of the heat dissipation layer, to form a heating layer.

In an implementation, a viscosity of the second paste is greater than the viscosity of the first paste. In other words, the viscosity of the first paste is less than the viscosity of the second paste, so that the first paste has better flowability. The heat dissipation layer 12 formed by the first paste can fill defects on the surface of the porous substrate 11. Surface flatness of the formed heat dissipation layer 12 is high, thereby providing a flatter surface for the heating layer 13. The heating layer 13 is formed on the surface with a high degree of flatness, improving a product resistance value yield of the heating layer 13, and improving consistency of a resistance value of the heating layer 13.

In an implementation, the viscosity of the second paste is in a range of 400000 cp to 800000 cp. The second paste includes at least one of a nickel-based alloy paste, an iron-based alloy paste, and a ruthenium-based alloy paste.

The heating element 10 prepared in the preparation method for a heating element 10 provided in the foregoing embodiments is prepared in a simple manner, a binding force between the heat dissipation layer 12 and the porous substrate 11 is improved, the product resistance value yield of the heating layer 13 and the consistency of the resistance value are improved, and possibility of adaptability of the heating element 10 to different aerosol-forming materials is expanded.

This application further experimentally compares a heating element in the related art with the heating element 10 provided in the embodiments of this application. The heating element in the related art includes only a heating layer, and no heat dissipation layer is disposed. The line width of the heating layer is 0.4 mm and the line length of the heating layer is 8.6 cm, and the heating element is denoted as a first experimental member (where FIG. 6 is a schematic diagram of a product of a first experimental member). An experiment is performed by using a structure of the heating element 10 described in the foregoing embodiment. The line width of the heat dissipation layer 12 is 0.7 mm, the line width of the heating layer 13 is 0.4 mm, and the line length of the heating layer 13 is 8.6 cm. The line length of the heat dissipation layer 12 is the same as the line length of the heating layer 13, and the heating element 10 is denoted as a second experimental member (where FIG. 7 is a schematic diagram of a product of a second experimental member). An experiment is performed by using a structure of the heating element 10 described in the foregoing embodiment. The line width of the heat dissipation layer 12 is 0.7 mm, the line width of the heating layer 13 is 0.4 mm, and the line length of the heating layer 13 is 7.8 cm. The line length of the heat dissipation layer 12 is the same as the line length of the heating layer 13, and the heating element 10 is denoted as a third experimental member (where FIG. 8 is a schematic diagram of a product of a third experimental member). Performance of the porous substrate 11 of the second experimental member, performance of the heating layer 13 of the second experimental member, performance of the porous substrate 11 of the third experimental member, and performance of the heating layer 13 of the third experimental member are basically the same as performance of the substrate of the first experimental member and performance of the heating layer of the first experimental member.

Experiment 1: comparison of performance.

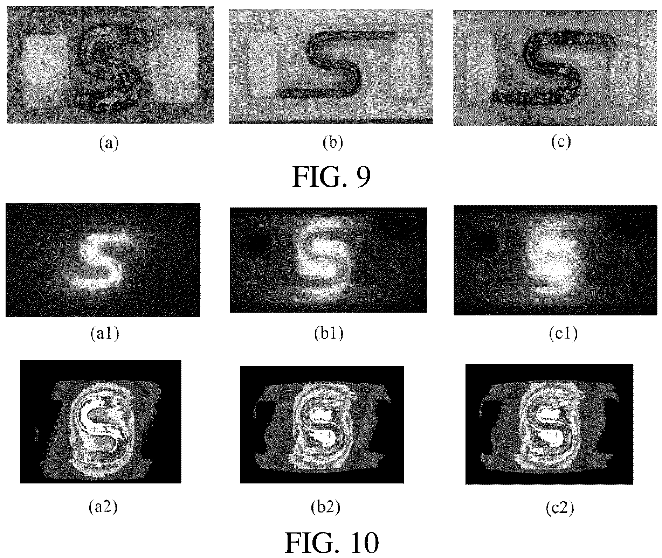

Test condition: each puff lasts 3s at an interval of 8s, and a device is tested according to a laboratory standard.
1. Dry heating service life at 6.5 W. The first experimental member can inhale 10 puffs to 15 puffs, the second experimental member can inhale 25 puffs to 30 puffs, and the third experimental member can inhale 25 puffs to 35 puffs.
2. Service life of a die being wet heated. The first experimental member can inhale 400 puffs to 600 puffs, the second experimental member can inhale more than 1500 puffs, and the third experimental member can inhale more than 1500 puffs.
3. Installation performance (7.5 W, material 1). That is, the heating element is assembled as an atomizer for testing.
   (1) Service life. The first experimental member can inhale 400 puffs to 600 puffs, the second experimental member can inhale more than 1000 puffs, and the third experimental member can inhale more than 1000 puffs.
   (2) Atomization amount. An atomization amount of the first experimental member is 7.0 mg, an atomization amount of the second experimental member is 7.5 mg, and an atomization amount of the third experimental member is 7.8 mg.
   (3) Fouling status after 500 puffs are inhaled. A fouling status after the first experimental member inhales 500 puffs is shown in FIG. 9. FIG. 9 is a comparison diagram of fouling after a first experimental member, a second experimental member, and a third experimental member separately inhale 500 puffs under the material 1. It should be noted that, in FIG. 9, (a) is the first experimental member, (b) is the second experimental member, and (c) is the third experimental member.

It can be learned from the foregoing experiments that, a dry heating service life, a service life of a die being wet heated, and installation performance of both the second experimental member and the third experimental member are significantly better than those of the first experimental member. It can be learned from comparison between the second experimental member and the third experimental member that, an atomization amount can be further increased by shortening the line length.

Experiment 2: comparison of temperature field performance.

FIG. 10 is a comparison diagram of temperature fields of a first experimental member, a second experimental member, and a third experimental member. It should be noted that, in FIG. 10, (a1) is a temperature field in infrared of the first experimental member, (a2) is a temperature distribution diagram of the first experimental member, where different colors represent different temperatures, (b1) is a temperature field in infrared of the second experimental member, (b2) is a temperature distribution diagram of the second experimental member, (c1) is a temperature field in infrared of the third experimental member, and (c2) is a temperature distribution diagram of the third experimental member.

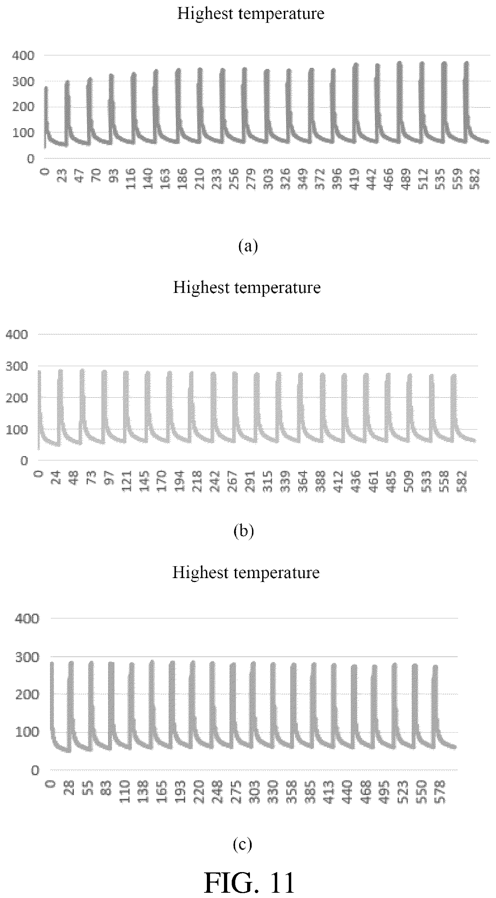

FIG. 11 is a curve diagram of highest temperatures of a first experimental member, a second experimental member, and a third experimental member. It should be noted that, in FIG. 11, (a) is the first experimental member, (b) is the second experimental member, and (c) is the third experimental member.

It can be learned from the foregoing experiments that, a high temperature region of the first experimental member is concentrated on a heating layer. A temperature field of the second experimental member and a temperature field of the third experimental member are concentrated in a quadrilateral region surrounded and formed by an edge of the heating layer. A highest temperature region is concentrated in a blank region defined and formed by an S-shaped film, and there is a larger and more uniform high temperature region, to avoid concentration of a high temperature on the heating layer, and reduce fouling on the heating layer. In this way, the second experimental member and the third experimental member perform more well, and stability and consistency of temperature fields of the second experimental member and the third experimental member are relatively high, and the temperature fields are stably controlled to be lower than 300 °C, which is consistent with a temperature in the national standard. A taste is consistent, and a service life is increased.

Experiment 3: comparison of binding forces.
1. Performance of an infiltration depth.

An infiltration depth surface test is separately performed by using the first experimental member and the second experimental member, and a plurality of parts of the first experimental member and the second experimental member are compared. Test results are shown in FIG. 12. FIG. 12 is a comparison diagram of infiltration of a first experimental member and a second experimental member. It should be noted that, in FIG. 12, (a1) is a representation diagram of an infiltration depth of a part A of the first experimental member, (a2) is a representation diagram of an infiltration depth of a part B of the first experimental member, (a3) is a representation diagram of an infiltration depth of a part C of the first experimental member, (b1) is a representation diagram of an infiltration depth of a part D of the second experimental member, (b2) is a representation diagram of an infiltration depth of a part E of the second experimental member, and (b3) is a representation diagram of an infiltration depth of a part F of the second experimental member. The part A of the first experimental member corresponds to the part D of the second experimental member, the part B of the first experimental member corresponds to the part E of the second experimental member, and the part C of the first experimental member corresponds to the part F of the second experimental member.

An infiltration depth of the first experimental member is in a range of 50 µm to 80 µm, and an infiltration depth of the second experimental member is in a range of 100 µm to 140 µm.

2. Binding force test. A force value of the first experimental member is 75 MPa±25 MPa, a force value of the second experimental member is 135 MPa±15 MPa, and a force value of the third experimental member is 125 MPa±15 MPa.

Through comparison of the binding forces, it is found that, a heat dissipation layer 12 is disposed between a porous substrate 11 and a heating layer 13, so that a binding force between the heating layer 13 and the porous substrate 11 can be greatly improved, and a risk of disengagement and tilt of the heating layer 13 is greatly reduced, thereby increasing a service life of the heating element 10.

Experiment 4: fouling test. For a test condition, refer to the "installation performance" part in Experiment 1.

FIG. 13 is a comparison diagram of fouling after inhaling of a first experimental member, a second experimental member, and a third experimental member. It should be noted that, in FIG. 13, (a1) is a product diagram before the first experimental member performs inhaling, (a2) is a product diagram after the first experimental member inhales a material 2, (a3) is a product diagram after the first experimental member inhales a material 3, (b1) is a product diagram before the second experimental member performs inhaling, (b2) is a product diagram after the second experimental member inhales the material 2, (b3) is a product diagram after the second experimental member inhales the material 3, (c1) is a product diagram before the third experimental member performs inhaling, (c2) is a product diagram after the third experimental member inhales the material 2, and (c3) is a product diagram after the third experimental member inhales the material 3. For example, the material 3 is a sweet aerosol-forming material.

Through the foregoing comparison of fouling after inhaling, under the same condition, there is almost no fouling after the second experimental member and the third experimental member perform inhaling, indicating a higher application value of a quantity of puffs. A heat dissipation layer 12 is disposed between a porous substrate 11 and a heating layer 13, so that fouling on the heating layer 13 can be reduced.

Experiment 5: comparison of a degree of flatness.

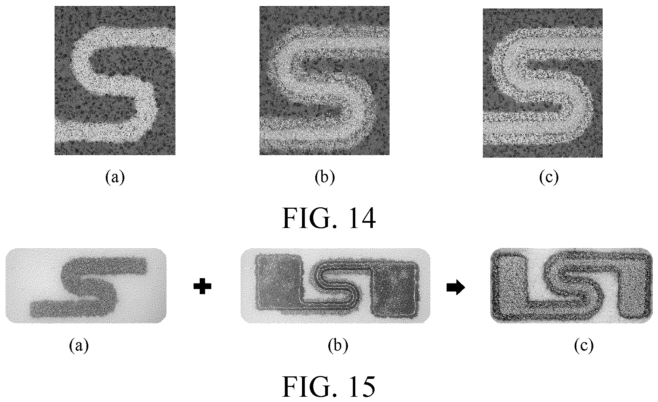

FIG. 14 is a comparison diagram of a degree of flatness of a first experimental member, a second experimental member, and a third experimental member. It should be noted that, in FIG. 14, (a) is the first experimental member, (b) is the second experimental member, and (c) is the third experimental member.

An edge of a heating layer of the first experimental member is flash. An edge of a heating layer 13 of the second experimental member and an edge of a heating layer 13 of the third experimental part are relatively flat and have a relatively high degree of flatness. In this way, along an extension direction of the heating layer 13, consistency of resistances of the heating layer 13 is improved, and consistency of an overall resistance value of a heating element 10 product is relatively high.

FIG. 15 is a flowchart of a preparation product of a second experimental member. It should be noted that, in FIG. 15, (a) is a product diagram in which the second experimental member is screen-printed to form a heat dissipation layer 12, (b) is a product diagram in which the second experimental member is screen-printed to form the heat dissipation layer 12 and a heating layer 13, and (c) is a product diagram in which the second experimental member is formed after the heat dissipation layer and the heating layer are co-heated. An edge of the heating layer 13 of the second experimental member is relatively flat, and a degree of flatness is relatively high.

An embodiment of this application provides a heating element 10, including: a porous substrate 11, a heat dissipation layer 12, and a heating layer 13. The heat dissipation layer 12 is disposed on the porous substrate 11, where the heat dissipation layer 12 has a porous structure; and the heating layer 13 is disposed on a surface of the heat dissipation layer 12 away from the porous substrate 11. The heat dissipation layer 12 has the porous structure, so that a liquid supply channel is provided to the heating layer 13, to maintain sufficient liquid supply to the heating layer 13 and a continuous atomization. The heat dissipation layer 12 is disposed between the porous substrate 11 and the heating layer 13, so that the heat dissipation layer 12 in contact with the porous substrate 11 can quickly conduct heat to a region of the porous substrate 11 that is not covered by the heat dissipation layer 12, and an aerosol-forming material stored in the region of the porous substrate 11 that is not covered by the heat dissipation layer 12 can be quickly atomized. Based on a fact that the same amount of atomization is implemented, an atomization temperature of the heating element 10 can be reduced, to improve a sense of use experience of a user.

The foregoing descriptions are merely implementations of this application, and the protection scope of this application is not limited thereto. All equivalent structure or process changes made according to the content of this specification and accompanying drawings in this application or by directly or indirectly applying this application in other related technical fields shall fall within the protection scope of this application.

## Claims

1. A heating element (10), **characterized by** comprising:
a porous substrate (11);
a heat dissipation layer (12), disposed on the porous substrate (11), wherein the heat dissipation layer (12) has a porous structure; and
a heating layer (13), disposed on a surface of the heat dissipation layer (12) away from the porous substrate (11).

2. The heating element (10) of claim 1, wherein a thickness of the heat dissipation layer (12) is less than a thickness of the heating layer (13); and/or
a porosity of the heat dissipation layer (12) is greater than a porosity of the heating layer (13); and/or
the heat dissipation layer (12) and the heating layer (13) each have a strip-shaped structure, and a line width of the heat dissipation layer (12) is greater than a line width of the heating layer (13).

3. The heating element (10) of claim 2, wherein
the porosity of the heat dissipation layer (12) is in a range of 30% to 70%; and/or
the line width of the heat dissipation layer (12) is in a range of 0.5 mm to 0.8 mm; and/or
a resistance of the heat dissipation layer (12) is in a range of 8 S2 to 20 Ω; and/or
the thickness of the heating layer (13) is in a range of 40 µm to 100 µm; and/or
the porosity of the heating layer (13) is in a range of 0 to 10%; and/or
the line width of the heating layer (13) is in a range of 0.25 mm to 0.45 mm; and/or
a resistance of the heating layer (13) is in a range of 0.85 S2 to 1.60 Ω.

4. The heating element (10) of claim 1, wherein an orthographic projection of the heat dissipation layer (12) on the heating layer (13) completely covers the heating layer (13).

5. The heating element (10) of claim 4, wherein an orthographic projection of a center line of the heat dissipation layer (12) along an extension direction on the heating layer (13) coincides with a center line of the heating layer (13) along the extension direction.

6. The heating element (10) of claim 1, wherein the porous substrate (11) comprises an atomization surface (111); the heat dissipation layer (12) comprises a main body portion (121) and an infiltration portion (122), the main body portion (121) is disposed on the atomization surface (111) of the porous substrate (11), and the infiltration portion (122) is disposed in holes of the porous substrate (11); and

7. The heating element (10) of claim 6, wherein a thickness (D) of the infiltration portion (122) is in a range of 80 µm to 150 µm; and/or a thickness of the main body portion (121) is in a range of 5 µm to 20 µm.

8. The heating element (10) of claim 1, wherein a material of the heating layer (13) comprises at least one of a nickel-based alloy, an iron-based alloy, and a ruthenium-based alloy; and/or
a material of the heat dissipation layer (12) comprises at least one of the nickel-based alloy, the iron-based alloy, and the ruthenium-based alloy.

9. The heating element (10) of claim 1, wherein the heating layer (13) and the heat dissipation layer (12) share the same shape and both extend in a curve.

10. The heating element (10) of claim 1, wherein a line length of the heat dissipation layer (12) is the same as a line length of the heating layer (13).

11. An atomizer (1), **characterized by** comprising a liquid storage cavity and the heating element (10) of any one of claims 1 to 10, wherein the liquid storage cavity is configured to store an aerosol-forming material, and the heating element (10) is configured to heat and atomize the aerosol-forming material in the liquid storage cavity.

12. An electronic atomization device, **characterized by** comprising a main unit (2) and the atomizer (1) of claim 8, wherein the main unit (2) is configured to supply power to the atomizer (1).

13. A preparation method for a heating element, **characterized by** comprising:
providing (S01) a porous substrate (11), wherein the porous substrate (11) comprises an atomization surface (111);
coating (S02) a first paste on the atomization surface (111) of the porous substrate (11), to form a heat dissipation layer (12); and
coating (S03) a second paste on a surface of the heat dissipation layer (12), to form a heating layer (13).

14. The preparation method of claim 13, wherein a viscosity of the first paste is less than a viscosity of the second paste.

15. The preparation method of claim 14, where the viscosity of the first paste is in a range of 50000 cp to 150000 cp; and/or the viscosity of the second paste is in a range of 400000 cp to 800000 cp.
